(19) Europäisches Patentamt
European Patent Office
Office européen des brevets

(11) EP 3 216 441 A1

(12) EUROPEAN PATENT APPLICATION

(43) Date of publication:
**13.09.2017 Bulletin 2017/37**

(51) Int Cl.:
***A61K 8/11** (2006.01)* ***A61K 8/73** (2006.01)*
***A61Q 15/00** (2006.01)*

(21) Application number: **16382105.1**

(22) Date of filing: **09.03.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **La Superquimica, S.A.**
**08907 Hospitalet de Llobregat (Barcelona) (ES)**

(72) Inventors:
- **ERRA LOZANO, Marina**
**08907 L'HOSPITALET DE LLOBREGAT (ES)**
- **FLORIACH GUAL, Nuria**
**08907 L'HOSPITALET DE LLOBREGAT (ES)**
- **RUANO ALDEA, Marta**
**08225 TERRASSA (ES)**

(74) Representative: **ZBM Patents - Zea, Barlocci & Markvardsen**
**Plaza Catalunya, 1 2nd floor**
**08002 Barcelona (ES)**

# (54) A ANTIPERSPIRANT COMPOSITION HAVING LONG-LASTING ODOR PROTECTION

(57) The present invention relates a genipin cross-linked alginate-chitosan microcapsule comprising an effective amount of one or more encapsulated antiperspirant active ingredients, wherein the microcapsule has a percentage of croslinking comprised from 20 to 40% expressed in absorbance values, to topical compositions containing them; processes for their preparations as well as their use as an antiperspirant and deodorant agents.



Printed by Jouve, 75001 PARIS (FR)

## Description

The present invention relates to the antiperspirant and deodorant field. In particular to a non-irritanting antiperspirant and deodorant genipine cross-linked alginate-chitosan microcapsules having one or more encapsulated antiperspirant active agents, to compositions containing them. It also relates to processes for their preparation and their uses as antiperspirant and deodorant agent.

BACKGROUND ART

Antiperspirant and deodorant compositions are popular personal care products used to prevent or eliminate perspiration and body odor caused by perspiration. Most of antiperspirant compositions commonly used include at least one antiperspirant active ingredient that minimize or prevent the secretion of perspiration by blocking or plugging ducts of sweat-secreting glands. Further, most of the deodorant compositions commonly used include at least one deodorant active agents that minimize, prevent and/or eliminate malodor associated with perspiration.
These antiperspirant and deodorant compositions typically used are effective to a greater or lesser extent, but mostly have an effect lasting less than a number of hours, and typically no more than 24 hours and often considerably less.
Antiperspirant actives ingredients typically used are aluminum-containing and/or zirconium-containing salts; preferably aluminum-containing salts of strong inorganic acids. These salts upon contact with perspiration enter the sweat duct via diffusion or convection and react with the basic components of perspiration blocking the perspiration. However, such compounds frequently cause irritation upon contact with the skin, in some cases producing dermatitis. These antiperspirant active agents also attack cellulosic fabrics due to liberation of strong acids from the salts. Due to this fact, the antiperspirant industry has migrated away from the use of these antiperspirant active ingredients.
Less or non-irritating antiperspirant active ingredients have been developed.
Unfortunately, most of these compositions that have been used to reduce skin irritation also significantly reduce the antiperspirant efficacy and/or the long lasting antiperspirant effect. Particularly, the use of aluminum-containing salts of weaker acids as non-irritaing antiperspirant active ingredients has been disclosed. However, these compounds have proved much less effective in inhibiting perspiration and they also do not prevent odors as well as the salts of strong acids traditionally used.
Thus, from what is known in the art it is derived that there is still the need of developing a non-irritating antiperspirant and deodorant compositions having a long-lasting antiperspirant and odor protection.

SUMMARY OF THE INVENTION

Inventors have found that a genipine cross-linked alginate-chitosan microcapsule which comprises an effective amount of one or more encapsulated antiperspirant active ingredients, wherein the microcapsule has a percentage of croslinking comprised from 20 to 40% expressed in absorbance values exhibit a long lasting protection up to 72 hours without provoking skin irratation in the area of application. Besides, the microcapsules of the present invention which comprises chitosan allows have a dual activity as an antiperspirant and deodorant; and also allows reducing the therapeutic affective amount of antiperspirant active agent without compromizing the antiperspirant and deodorant effect.
Furthermore, the microcapsules of the present invention are also advantageous because they have the appropriate stability for being included in all types of antiperspirant and deodorant compositions commonly used without compromizing the controlled release of the antiperspirant active ingredient which is encapsulated inside the microcapsule.
Thus, an aspect of the present invention refers to a genipin cross-linked alginate-chitosan microcapsule comprising an effective amount of one or more encapsulated antiperspirant active ingredients, wherein the microcapsule has a percentage of croslinking comprised from 20 to 40% expressed in absorbance values.
The second aspect of the invention refers to a process for the preparation of the genipin cross-linked alginate-chitosan microcapsule as defined in the first aspect of the invention, which comprises: i) preparing an acidic aqueous solution of the chitosan by contacting an acidic aqueous solution with chitosan; ii) adding the encapsulated antiperspirant active ingredient to the solution obtained in step (i) to obtain the solution A; iii) preparing an aqueous solution of the alginate and the genipin to obtain solution B; iv) adding the solution B to the solution A and mixing the resulting emulsion under a period of time comprised from 1 to 6 hours to obtain a wet genipin cross-linked alginate-chitosan microcapsule comprising an effective amount of one or more encapsulated antiperspirant active ingredients; and v) spray-drying the wet microcapsule obtained in step (iv).
The third aspect of the invention refers to a topical pharmaceutical or cosmetic composition comprising an effective amount of genipin cross-linked alginate-chitosan microcapsules as defined in the first aspect of the invention, together with appropriate topical pharmaceutically or cosmetically acceptable excipients or carriers.
The fourth aspect of the invention refers to the use of genipine cross-linked alginate-chitosan microcapsules as defined in the first aspect of the invention, or alternatively the topical pharmaceutical or cosmetic composition as

defined in the third aspect of the invention as an antiperspirant and deodorant agent.

BRIEF DESCRIPTION OF THE DRAWINGS

FIG. 1 section A shows the inner structure of the genipine cross-linked alginate-chitosan microcapsule of the present invention and section B shows the cross-links of genipin between the chains of chitosane. Ch represents the bi-layer of chitosan chains forming part of the external layer of the microcapsules; G represents the cross-links of genipine that links the two layers of chitosan; and A represents the alginate chains forming part of the inner layer of the microcapsules.

FIG. 2 shows the SEM image (Scanning Electronical Microscopy image) at magnification of 500X. Section (A) shows the intact microcapsules of Example 1 after being submitted during three months at a 40°C; and section (B) shows the broken microcapsules of Comparative Example 1 after being submitted during three months at a 25°C.

DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the invention, any ranges given include both the lower and the upper end-points of the range. Ranges given, such as temperatures, times, and the like, should be considered approximate, unless specifically stated.

The term "percentage (%) by weight" refers to the percentage of each ingredient of the microcapsule or composition in relation to the total weight.

The term "percentage (%) of crosslinking" refers herein is the percentage of cross-links of genipin of the microcapsules of the invention expressed in absorbance values. This value is calculated by the following equation:

$$(\%)\ \text{of crosslinking} = ([A_{nx} - A_x]/A_{nx}) \times 100$$

wherein $A_{nx}$ is the absorbance values for the free amino groups in the non crosslinked microcapsules, and $A_x$ is the absorbance values for the free amino groups in the genipin cross-linked alginate-chitosan microcapsule.

The optical absorbances were measured at a temperature of 25°C and at a wavelenght of 570 nm with an UV-Vis spectrophotometer (Perkin Elmer) using glycine solution for the standard curve. This determination is carried out from absorbance measurements using the Beer-Lambert Law (cf. Schmid, F.X. "Optical spectroscopy to characterize protein conformation and conformational changes. In Protein Structure: A Practical Approach", T.E. Creighton, ed., IRL Press, Oxford, U.K., 1997, pp. 261-297) in the ninhydrin assay (Mi, F. L., Tan, Y. C., Liang, H. C., Huang, R. N., & Sung, H. W. "In vitro evaluation of a chitosan membrane cross-linked with genipin". Journal of Biomaterials Science Polymer Edition, 2001, vol. 12(8), pp. 835-850) using a UV Spectroscopy (Perkin Elmer).

The process for measuring the absorbance values comprises:

1) Preparation of a standard curve:

Prepare known concentrations of glycine solution (0.2-1mL). Add distilled water to all test tubes to make volume up to 4ml. Add 1 ml of Ninhydrin solution to all test tubes, vortex test tubes and place in boiling water bath for 15-20minutes. Cool to room temperature and add 1ml of ethanol solution to each test tube. Set the spectrophotometer at 570nm and build the curve Absorbance versus concentration at 570 nm. Zero Absorbance corresponds with a blank made of 4ml distilled water and 1 ml Ninhydrin solution. The determination of constant (K) is carried out by the following formula:

$$\text{Aglycine} = K \cdot \text{cglycine}$$

being A the absorbance values of the glycine and being c the concentration of glycine;

2) Preparation of the Ninhydrin solution:

Preparation of solution A: 1.05g citric acid, 10mL (1.0 M) NaOH and 0.04g $SnCl_2$ x $2H_2O$ were mixed, then deionized $H_2O$ added until 25mL;
Preparation of solution B: 1g ninhydrin was added to 25mL ethylene glycol monomethyl ether.
The two solutions A and B were combined and stirred for 45min, then stored in dark bottle.

3) Determination of the Absorbances ($A_{nx}$ and $A_x$):

Determination of $A_{nx}$: 10mg of the non crosslinked alginate-chitosan microcapsules were added to 4 mL of ninhydrin solution (pH3.5), and were maintained for 20min at 100°C. After that time, the non crosslinked alginate-chitosan microcapsules were separated by centrifugation. The solution was cooled down to room temperature, diluted with 5mL 50%v isopropanol, and then the optical absorbance $A_{nx}$ of the solution at 570nm was read.

Determination of $A_x$: 10mg of the genipin crosslinked alginate-chitosan microcapsules were added to 4 mL of ninhydrin solution (pH3.5), and were maintained for 20min at 100°C. After that time, genipin cross-linked alginate-chitosan microcapsules were separated by centrifugation. The solution was cooled down to room temperature, diluted with 5mL 50%v isopropanol, and then the optical absorbance of the solution at 570nm was read.

The term "weight ratio" refers to the relation of weights of two ingredients needed to promote the therapeutic or cosmetic effect after its application. Particularly, the relation of weights of chitosane and the antiperspirant active ingredient; or the relation of chitosan and alginate needed to promote the long lasting antiperspirant and deodorant effect without skin irritation.

The term "particle size" refers to the size of the microparticles measured in μm. The measurement was performed with an appropriate apparatus by conventional analytical techniques such as, for example, microscopic determination utilizing a scanning electron microscope (SEM). In the present invention the particle size was measured by a Mastersizer 2000 particle size analyzer. Such apparatus uses a technique of laser diffraction to measure the size of particles. It operates by measuring the intensity of light scattered, as a laser beam passes through a dispersed particles sample. This data is then analyzed using the general purpose model to calculate the size of the particles that created the scattering pattern, assuming a spherical particle shape.

The terms "particle size distribution" or "PSD" have the same meaning and are used interchangeably. They refer to the percentage of the microparticles within a certain size range. The term "D90" refers to the value of particle size distribution where at least 90% of the microparticles have a size less or equal to the given value. Further, the term "D50" refers to the value of particle size distribution where at least 50% of the particles have a size less or equal to the given value.

The term "pharmaceutically acceptable" refers to that excipients or carriers suitable for use in the pharmaceutical technology for preparing compositions with medical use.

The terms "cosmetically acceptable" or "dermatological acceptable" have the same meaning and which are herein used interchangeably. They refer to that excipients or carriers suitable for use in contact with human skin without undue toxicity, incompatibility, instability, allergic response, among others.

The term an "effective amount of the antiperspirant active ingredient" refers to the amount of the antiperspirant active ingredient that provides the therapeutic and/or cosmetic effect after its application on the skin (that is an antiperspirant and/or deodorant effect). The term an "effective amount of the microcapsule" refers to the amount of microcapsules which include the effective amount of the antiperspirant active ingredient that provide the therapeutic or cosmetic effect after its application on the skin.

The terms "genipine cross-linked alginate-chitosan microcapsule" or "GCAC" have the same meaning and are used interchangable. They refers to a microcapsule composed of an external layers fromed by two chains of chiosan crosslinked by genipin chains and an internal layer formed by alginate. The inner structure of genipine cross-linked alginate-chitosan microcapsule of the present invention is shown in Fig. 1.

The terms "genipine" or "genipin" have the same meaning and which are herein used interchangeably. They are the International Nonproprietary Name (INN) of methyl (1*R*,2*R*,6*S*)-2-hydroxy-9-(hydroxymethyl)-3-oxabicyclo[4.3.0] nona-4,8-diene-5-carboxylate which is an iridoid glycoside present in fruits such as Gardenia jasmindides Ellis. Its chemical structure corresponds to the following formula:

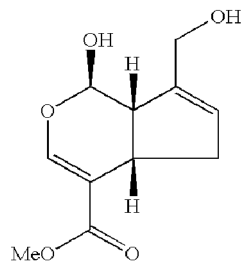

The terms "alginic acids" or "algin" or "alginate" have the same meaning and are used interchangable. They refers to a linear copolymer with homopolymeric blocks of (1-4)-linked β-D-mannuronate (M) and its C-5 epimer α-L-guluronate (G) residues, respectively, covalently linked together in different sequences or blocks. The monomers can appear in homopolymeric blocks of consecutive G-residues (G-blocks), consecutive M-residues (M-blocks) or alternating M and G-residues (MG-blocks). Its chemical structure corresponds to the following formula:

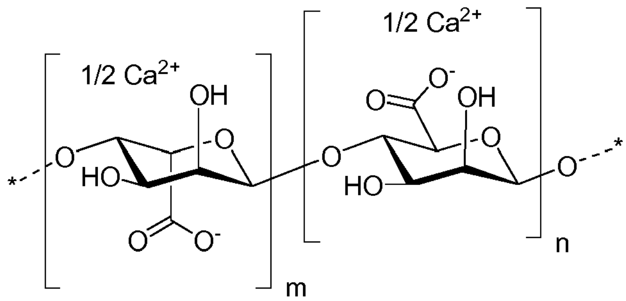

The term "chitosan" refers to a linear polysacharide composed of randomly distributed β-(1-4)- linked D-glucosamine and N-acetly-D-glucosamine. It is biodegradable and biocompatible and it is able to form a polyion complex with anionic polymers. Its chemical structure corresponds to the following formula:

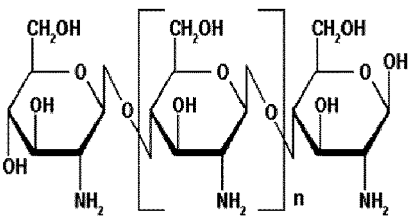

The terms "cross-linked" or "cross-linking" refers in the synthetic polymer science field, the use of cross-links to promote a difference in the physical properties of the polymers. The term "cross-links" refers to bonds that link one polymer chain to another or differents parts of the same polymer and these bonds can be covalent or ionic bonds. The term "cross-linker" or "crosslinking agent" refers to compound having the ability to cross-link the polymer chains. In the present invention, the term "percentage of crosslinking" refers to the amount of cross-links of genipin that link the bi-layer of chitosan expressed in percentage by weight of the weight of the microcapsule. The links between genipin and chitosan are created by reacting the primary amine group of the chitosan chains with the oxygen atoms of the genipin to obtain ester groups and 1, 2, 3, 4-tetrahydropyridine rings (cf. Fig 1).

In an embodiment, optionally in combination with one or more features of the various embodiments described above or below, the genipin cross-linked alginate-chitosan microcapsule of the invention is that which comprises a percentage of croslinking comprised from 22% to 37% expressed in absorbance values. In a preferred embodiment, optionally in combination with one or more features of the various embodiments described above or below, the genipin cross-linked alginate-chitosan microcapsule of the invention is that which comprises a percentage of croslinking comprised from 25% to 35% expressed in absorbance values; more preferably comprised from 28% and 31% expressed in absorbance values. It is advantageous because these microcapsules having the above mentioned percentage of crosslinking are stable when they are included in any typically used antiperspirant and/or deodorant composition.

In an embodiment, optionally in combination with one or more features of the various embodiments described above or below, the genipin cross-linked alginate-chitosan microcapsule is that wherein: the amount of genipin is comprised from 0.001 % to 5 % by weight of the weight of the microcapsule; the amount of the alginate is comprised from

1 % to 20 % by weight of the weight of the microcapsule; the amount of chitosan is comprised from 1% to 20% by weight of the weight of the microcapsule; the weight ratio between the chitosan and the encapsulated antiperspirant active ingredient salt is comprised from 1:2 to 1:50; and the weight ratio between the chitosan and alginate is comprised from 1:1 to 4:1. In a preferably embodiment, optionally in combination with one or more features of the various embodiments described above or below, the genipin cross-linked alginate-chitosan microcapsule is that wherein: the amount of genipin is comprised from 0.001 % to 3 % by weight of the weight of the microcapsule; the amount of the alginate is comprised from 1 % to 10 % by weight of the weight of the microcapsule; the amount of chitosan is comprised from 1 % to 10% by weight of the weight of the microcapsule; the weight ratio between the chitosan and the encapsulated antiperspirant active ingredient salt is comprised from 1:5 to 1:50; and the weight ratio between the chitosan and alginate is comprised from 1:1 to 4:1

In an embodiment, optionally in combination with one or more features of the various embodiments described above or below, the genipin cross-linked alginate-chitosan microcapsule is that wherein: the amount of genipin is comprised from 0.001 % to 1 % by weight of the weight of the microcapsule; the amount of alginate is comprised from 1 % to 3% by weight of the weight of the microcapsule; the amount of chitosan is comprised from 1.5% to 4% by weight of the weight of the microcapsule; the weight ratio between the chitosan and the encapsulated antiperspirant active ingredient salt is comprised from 1:10 to 1:40; and the weight ratio between the chitosan and alginate is comprised from 1:1 to 2:1.

In an embodiment, optionally in combination with one or more features of the various embodiments described above or below, the genipin cross-linked alginate-chitosan microcapsule is that wherein: the amount of genipin is 0.004% by weight of the weight of the microcapsule; the amount of alginate is 1.2% by weight of the weight of the microcapsule; the amount of chitosan is 2.4% by weight of the weight of the microcapsule; the weight ratio between the chitosan and the encapsulated antiperspirant active ingredient salt is 1:40; and the weight ratio between the chitosan and alginate is 2:1.

The genipin cross-linked alginate-chitosan microcapsule of the present invention comprises one or more encapsulated antiperspirant active ingredients. The term "antiperspirant" active ingredient refers to any compound having antiperspirant activity.

For the purposes of the invention, the term "encapsulated antiperspirant" active ingredient refers to any compound having antiperspirant activity that is inside the microcapsules of the invention. Appropriate antiperspirant active ingredients for the present invention may include astringent metallic salts, especially inorganic and organic salts of aluminum, zirconium and zinc, and mixture thereof. Even more specifically, the antiperspirant active ingredients may include aluminum-containing and/or zirconium-containing salts or materials, such as, for example, aluminum halides, aluminum chlorohydrate, aluminum hydroxyhalides, zirconyl oxyhalides, zirconyl hydroxyhalides, and mixtures thereof.

In an embodiment, optionally in combination with one or more features of the various embodiments described above or below, the encapsulated antiperspirant active ingredient is an aluminum-containing salt of formula:

$$Al_2(OH)_aCl_b.xH_2O$$

wherein:

- a is comprised from 2 to 5;
- the sum of a and b is 6;
- x is comprised from 1 to 6; and
- a, b, and x is selected from the group consisting of an integer or a non-integer value.

In an alternative embodiment, optionally in combination with one or more features of the various embodiments described above or below, the encapsulated antiperspirant active ingredient is a zirconium salt of formula:

$$ZrO(OH)_{2-a}Cl_a.xH_2O,$$

wherein:

- a is comprised from 1.5 to 1.87;
- x is comprised from 1 to 7; and
- a and x is selected from the group consisting of an integer or a non-integer value.

Examples of aluminum-containing salts appropriate for the present invention include, but are not limited to, hydrated aluminum chloride ($[Al_2(OH)5Cl]\ nH_2O$), hydrated sesqui hydrogen chloride alumina ($[Al_2(OH)4.5Cl_{1.5}]nH_2O$), dihydrochloride hydrated alumina ($[Al_2(OH)4Cl_2]nH_2O$), hydrogen chloride hydrated alumina (aluminum-containing salt chlorohydrex) propylene glycol (PG) or polyethylene glycol (PEG) ($[Al_2(OH)5Cl]nH_2O + CH_3CHOHCH_2OH$ or

$H(OCH)_2(CH_2)_2OH$), hydrated aluminum sesqui hydrogen chloride PG or PEG, aluminum PG or hydrated dihydrochloride PEG, and hydrated aluminum hydroxide ($Al(OH)3nH_2O$), aluminum hydroxide, zirconium chloride hydrated salt, such as hydrated aluminum zirconium trichlorosilane ($[Al_{3.8}Zr(OH)\ 12.4Cl_3]nH_2O$), hydrated aluminum hydroxide, zirconium tetrachloride ($[Al_3\ 6Zr(OH)11.6Cl_{3.2}]nH_2O$), pentachlorophenol hydrated zirconium hydroxide ($[Al_8Zr(OH)23Cl_5]nH_2O$), eight chlorine hydrate zirconium hydroxide ($[Al_8Zr\ (OH)20Cl_8]nH_2O$), hydrated trichlorosilane aluminum zirconium glycine ($[Al3.8Zr(OH)12.4Cl_3]nH_2O + H_2NCH_2COOH$), hydrated zirconium tetrachloride glycine aluminum hydroxide, hydrated zirconium glycine pentachlorophenol aluminum hydroxide, aluminum hydroxide, chlorine hydrate eight zirconium glycine, aluminum sulfate potassium salt ($KAl(SO_4)\ 212H_2O$), aluminum undecylenoyl collagen amino acids, aluminum lactate + aluminum sulfate ($Al_2(SO_4)_3 + Na_2HAl(OOCCHOHCH_3)_2$- (OH) 6), glycolic acid chloride sodium aluminum bromide, aluminium sesquichlorohydrate, aluminum hydroxide, hydrated ($Al_2Br(OH)5nH_2O$), hydrated aluminum ($AlCl_36H_2O$) chloride, complex compound of zinc and sodium, lanthanum and cerium complexes and fatty acids aluminum-containing salt.

In an embodiment, optionally in combination with one or more features of the various embodiments described above or below, the encapsulated aluminum-containing salt of the genipin cross-linked alginate-chitosan microcapsule of the invention is selected from the group consisting of aluminum halohydrate, aluminum-zirconium halohydrate, aluminum nitrohydrate compounds and mixture thereof. Preferably, optionally in combination with one or more features of the various embodiments described above or below, the encapsulated aluminum-containing salt of the genipin cross-linked alginate-chitosan microcapsule of the invention is aluminum sesquichlorohydrate.

In an embodiment, optionally in combination with one or more features of the various embodiments described above or below, the encapsulated antitranspirant active ingredient is an encapsulated aluminum-containing salt in an amount comprised from 92% to 98% by weight of the weight of the microcapsule.

In an embodiment, optionally in combination with one or more features of the various embodiments described above or below, the genipin cross-linked alginate-chitosan microcapsule of the present invention is that which has a particle size distribution D90 comprised from 10 μm to 80μm; preferably the particle size distribution D90 comprised from particle size distribution D90 comprised from 20 μm to 80μm.; more preferably, comprised from 20μm to 60μm.

Other aspect of the invention is a process for the preparation of the microcapsules of the first aspect of the invention. The process for the manufacturing of these microcapsules comprises: i) preparing an acidic aqueous solution of the chitosan by contacting an acidic aqueous solution with chitosan; ii) adding the aluminum-containing salt to the solution obtained in step (i) to obtain the solution A; iii) preparing an aqueous solution of the alginate and the genipin to obtain solution B; iv) adding the solution B to the solution A and mixing the resulting emulsion under a period of time comprised from 1 to 6 hours to obtain a wet genipin cross-linked alginate-chitosan microcapsule; and v) spray-drying the microcapsule obtained in step (iv). This process allows obtaining the stables microcapsules of the first aspect of the invention having the appropriate crosslinking percenatge to be stable in the commonly used antiperspirant compositions and allowing the prolonged release of the encapsulated antiperspirant active ingredient from the microcapsules of the invention to have a long antiperspirant and deodorant lasting effect.

In an embodiment, optionally in combination with one or more features of the various embodiments described above or below, the mixture of solution A and B in step (iv) is performed for a period of time comprised from 1 to 6 hours to obtain a wet genipin cross-linked alginate-chitosan microcapsule. The above mentioned period of time allows obtaining the genipin cross-linked alginate-chitosan microcapsules of the inveniton having a percentage of crosslinking comprised from 20% to 40%. In an embodiment, optionally in combination with one or more features of the various embodiments described above or below, wherein when the genipin cross-linked alginate-chitosan microcapsule are forming part of a spray composition, then the mixture of soluiton A and B in step (iv) is performed for a period of time comprised from 2 hours to 3 hours; preferably from 2.5 hours to 3 hours. In an alternative embodiment, optionally in combination with one or more features of the various embodiments described above or below, wherein when the genipin cross-linked alginate-chitosan microcapsule are forming part of a roll-on composition, then the mixture of solution A and B in step (iv) is performed for a period of time comprised from 4 hours to 6 hours; preferably from 4.5 hours to 5 hours.

In an embodiment, optionally in combination with one or more features of the various embodiments described above or below, step (v) is carrying out by spray drying wherein the inlet temperature is comprised from 170°C to 210°C; the outlet temperature is comprised from 102°C to 119°C; and the feed flow is comprised from 2 to 15 ml/min. In an embodiment, optionally in combination with one or more features of the various embodiments described above or below, step (v) is carrying out by spray drying wherein the inlet temperature is comprised from 190°C to 210°C; the outlet temperature is comprised from 102°C to 110°C; and the feed flow is comprised from 2 to 15 ml/min.

In an embodiment, optionally in combination with one or more features of the various embodiments described above or below, step (v) is carrying out by spray drying wherein the inlet temperature is comprised from 200°C; the outlet temperature is comprised from 104°C; and the feed flow is comprised from 2 to 3mL/min

In an embodiment, optionally in combination with one or more features of the various embodiments described above or below, the acidic aqueous solution of step (i) is a solution of an acid selected from the group consisting of acetic acid, propionic acid, and mixture thereof; preferably, the acidic aqueous solution of step (i) is an acetic aqueous

solution. In an embodiment, optionally in combination with one or more features of the various embodiments described above or below, the acidic aqueous solution of step (i) has a concentration comprised from 1 to 15g/L; preferably comprised from 1 to 10g/L. In an embodiment, optionally in combination with one or more features of the various embodiments described above or below, the acidic aqueous solution of step (i) is an acetic aqueous solution in a concentration comprised from 2.5 to 3g/L; preferably in a concentration of 5g/L.

The genipin cross-linked alginate-chitosan microcapsule of the invention may be defined by its preparation process as defined above and therefore, the genipin cross-linked alginate-chitosan microcapsule obtainable by the process above is considered part of the invention. Thus, the genipin cross-linked alginate-chitosan microcapsule defined above is obtainable by the process comprising: i) preparing an acidic aqueous solution of the chitosan by contacting an acidic aqueous solution with chitosan; ii) adding the aluminum-containing salt to the solution obtained in step (i) to obtain the solution A; iii) preparing an aqueous solution of the alginate and the genipin to obtain solution B; iv) adding the solution B to the solution A and mixing the resulting emulsion under a period of time comprised from 1 to 6 hours to obtain a wet genipin cross-linked alginate-chitosan microcapsule; and v) spray-drying the microcapsule obtained in step (iv) is also part of the invention.

For the purposes of the invention the expressions "obtainable", "obtained" and equivalent expressions are used interchangeably, and in any case, the expression "obtainable" encompasses the expression "obtained".

All the embodiments disclosed above for the process of the second aspect of the invention applies also for the genipin cross-linked alginate-chitosan microcapsule obtainable by this process.

The microcapsules of the present invention can be forming part of a topical pharmaceutical or cosmetic composition. Thus, the topical pharmaceutical or cosmetic composition of the present invention comprises an effective amount of the genipin cross-linked alginate-chitosan microcapsule as defined above together with one or more appropriate topical pharmaceutically or cosmetically acceptable excipients or carriers.

In a particular embodiment, the topical composition is a pharmaceutical composition comprising an effective amount of t the genipin cross-linked alginate-chitosan microcapsule as defined above together with one or more appropriate topical pharmaceutically acceptable excipients or carriers.

In another particular embodiment, the topical composition is a cosmetic composition comprising an effective amount of the genipin cross-linked alginate-chitosan microcapsule as defined above together with one or more appropriate topical cosmetically acceptable excipients or carriers.

The topical compositions of the invention can be formulated in several forms that include, but are not limited to, creams, powders, stick, roll-on, spray, soft solids, gels, mousses, ointments and pastes; preferably, the composition is in form of spray or roll-on.

Topical compositions of the present invention can be prepared according to methods well known in the state of the art. The appropriate excipients and/or carriers, and their amounts, can readily be determined by those skilled in the art according to the type of formulation being prepared.

The topical compositions defined above comprise appropriate excipients or carriers for topical administration that can be pharmaceutical or cosmetic excipients. When the composition is in form of spray, then the composition can include, but not limited to, a vehicle, emollient, surfactant, propellant and combinations thereof. When the composition is in form of roll-on, then the composition can include, but not limited to, a vehicle, emollient, surfactant, pH-regulating agent (buffering agent), preservatives and mixture thereof. The excipients or carriers used have affinity for the skin, are well tolerated, stable, and are used in an amount adequate to provide the desired consistency, and ease application.

In an embodiment, optionally in combination with one or more features of the various embodiments described above or below, wherein when the topical composition of the invention is in form of a spray, then the genipin cross-linked alginate-chitosan microcapsules as defined above has a percentage of crosslinking comprised from 20 to 30% by weight; preferably comprised from 25 to 30% by weight.

In an embodiment, optionally in combination with one or more features of the various embodiments described above or below, wherein when the topical composition of the invention is in form of a roll-on, then the genipin cross-linked alginate-chitosan microcapsules as defined above has a percentage of crosslinking comprised from 30 to 40% by weight; preferably comprised from 30 to 35% by weight.

The compositions mentioned above can include a vehicle. Examples of vehicles include, but are not limited to, water, propylene glycol, butylene glycol, alcohol such as ethanol, isopropanol, or silicones. In an embodiment of the invention, optionally in combination with one or more features of the various embodiments described above or below, when the composition of the invention is in form of spray, then the vehicle is selected from the group consisting of ethanol, cyclomethicone and mixture thereof; preferably cyclomethicone. In an alternative embodiment, optionally in combination with one or more features of the various embodiments described above or below, when the composition of the invention is in form of roll-on, then the vehicle is selected from the group consisting water, cyclomethicone, and mixture thereof; preferably water. The amount of vehicle in the compositions of the present invention is comprised from 5 to 20%.

The term "propellant" refers to one or more gases that are used to pressurize the antiperspirant composition to facilitate egress of the antiperspirant composition from the container. Some propellants may be a mixture of gases

(e.g., A-46 which is a mixture of isobutane, butane and propane). A propellant may be in the form of a liquid (i.e., a liquefied gas) when under pressure within the reservoir of a spray device. In addition, a propellant may be in its gaseous state within the head space of the reservoir. A propellant may be present in both a liquefied form and its gaseous state within the reservoir. Unless specified otherwise (e.g., liquid propellant or gaseous propellant), the term propellant is intended to encompass the liquefied form and the gaseous state individually and collectively. Example of appropriate propellant include, but are not limited to, compressed air, nitrogen, inert gases, carbon dioxide, and mixtures thereof. Propellants may also include gaseous hydrocarbons like propane, n-butane, isobutene, cyclopropane, and mixtures thereof. Halogenated hydrocarbons like 1,1-difluoroethane may also be used as propellants. Some non-limiting examples of propellants include 1,1,1,2,2-pentafluoroethane, 1,1,1,2-tetrafluoroethane, 1,1,1,2,3,3,3-heptafluoropropane, trans-1,3,3,3-tetrafluoroprop-1-ene, dimethyl ether, dichlorodifluoromethane (propellant 12), 1,1-dichloro-1,1,2,2-tetrafluoroethane (propellant 114), 1-chloro-1,1-difluoro-2,2-trifluoroethane (propellant 115), 1-chloro-1,1-difluoroethylene (propellant 142B), 1,1-difluoroethane (propellant 152A), monochlorodifluoromethane, and mixtures thereof. Some other propellants suitable for use include, but are not limited to, A-46 (a mixture of isobutane, butane and propane), A-31 (isobutane), A-17 (n-butane), A-108 (propane), AP70 (a mixture of propane, isobutane and n-butane), AP40 (a mixture of propane, isobutene and n-butane), AP30 (a mixture of propane, isobutane and n-butane), and 152A (1,1 difluoroethane). Preferably, the propellant is selected from the group consisting of butane, isobutane, propane, dimethylether and mixtures thereof. The amount of the propellant in the compositions of the present invention is comprised from 15 to 90% by weight.

The term "emollient" agent refers to a material that softens and soothes the skin in order to correct dryness and scaling of the skin, lubricating the skin surface, encouraging skin water retention, and altering product textures. Examples of appropriate topical emollient agents include, but are not limited to, propylene glycol, polypropylene glycol (like dipropylene glycol, tripropylene glycol, etc.), diethylene glycol, triethylene glycol, PEG-4, PEG-8, 1,2 pentanediol, 1,2 hexanediol, hexylene glycol, glycerin, C2 to C20 monohydric alcohols, C2 to C40 dihydric or polyhydric alcohols, alkyl ethers of polyhydric and monohydric alcohols, volatile silicone emollients such as cyclopentasiloxane, nonvolatile silicone emollients such as dimethicone, mineral oils, polydecenes, petrolatum, and combinations thereof. The amount of emollient agent in the compositions of the present invention is comprised from 1 to 95%.

The term "surfactant" refers to a material which lowers the surface tension of a liquid and the interfacial tension between two liquids, allowing their easier spreading. Surfactants have a hydrophilic head that is attracted to water molecules and a hydrophobic tail that repels water and simultaneously attaches itself to oil and grease in dirt. These opposing forces loosen the dirt and suspend it in the water, having the ability to remove it from surfaces such as the human skin, textiles, and other solids, when surfactants are dissolved in water. Examples of appropriate surfactant agents include, but are not limited to, nonionic surfactants, cationic surfactants, amphoteric surfactants, zwitterionic surfactants, and mixtures thereof. Examples of specific surfactants appropriate for the present invention include, but are not limited to, hydrogenated castor oil, polyoxyethylene 2 stearyl ether, polyoxyethylene 20 stearyl ether, and mixture thereof. One suitable hydrogenated castor oil that may be used in the present composition is polyoxyethylene hydrogenated castor oil. The amount of surfactant in the compositions of the present invention is comprised from 0.01% to 5%.

The term "preservative" refers to a material that prevents or reduces or slows down microbial growth, providing that the stability of the solution is not affected. Preferably, the preservative is any organic preservative material which will not cause damage to fabric appearance. Examples of appropriate preservative agents include, but are not limited to, water-soluble preservatives that include organic sulfur compounds, halogenated compounds, cyclic organic nitrogen compounds, low molecular weight aldehydes, parabens, propane diaol materials, isothiazolinones, quaternary compounds, benzoates, low molecular weight alcohols, dehydroacetic acid, phenyl and phenoxy compounds, or mixtures thereof. Non-limiting examples of commercially available water-soluble preservatives include a mixture of about 77% 5-chloro-2-methyl-4-isothiazolin-3-one and about 23% 2-methyl-4-isothiazolin-3-one, a broad spectrum preservative available as a 1.5% aqueous solution under the trade name Kathon® CG by Rohm and Haas Co.; 5-bromo-5-nitro-1,3-dioxane, available under the tradename Bronidox L® from Henkel; 2-bromo-2-nitropropane-1,3-diol, available under the trade name Bronopol® from Inolex; 1,1'-hexamethylene bis(5-(p-chlorophenyl)biguanide), commonly known as chlorhexidine, and its salts, e.g., with acetic and digluconic acids; a 95:5 mixture of 1,3-bis(hydroxymethyl)-5,5-dimethyl-2,4-imidazolidinedione and 3-butyl-2-iodopropynyl carbamate, available under the trade name Glydant Plus® from Lonza; N-[1,3-bis(hydroxymethyl)2,5-dioxo-4-imidazolidinyl]-N,N'-bis(hydroxymethyl)urea, commonly known as diazolidinyl urea, available under the trade name Germall® II from Sutton Laboratories, Inc.; N,N"-methylenebis{N'-[1-(hydroxymethyl)-2,5-dioxo-4-imidazolidinyl]urea}, commonly known as imidazolidinyl urea, available, e.g., under the trade name Abiol® from 3V-Sigma, Unicide U-13® from Induchem, Germall 115® from Sutton Laboratories, Inc.; polymethoxy bicyclic oxazolidine, available under the trade name Nuosept® C from Hüls America; formaldehyde; glutaraldehyde; polyaminopropyl biguanide, available under the trade name Cosmocil CQ® from ICI Americas, Inc., or under the trade name Mikrokill® from Brooks, Inc; dehydroacetic acid; and benzsiothiazolinone available under the trade name Koralone™ B-119 from Rohm and Hass Corporation. The amount of the preservatives in the compositions of the present invention is comprised from 0.0001 to 0.5%.

The term "thickening agent" or "thickener" or "viscosity agent" which is herein used interchangeably refers to a material that increases its viscosity without substantially modifying its other properties. Appropriate thickener include, but are not limited to, natural and synthetic or semi-synthetic thickeners which derive from various sources and consist of very different molecular structures including polysaccharides, proteins, alcohols, silicones or waxes. Examples of appropriate thickener include cetyl alcohol, stearyl alcohol, carnauba wax, and stearic acid, and cellulose derivatives such as hydroxyethylcellulose, guar gum, locust bean gum, xanthan gum, and gelatin.

The compositions of the present invention may contain other ingredients, such as fragrances, and other components known in the state of the art for use in topical formulations.

The composition of the present invention may contain additional active ingredients, such as for exemple deodorant agents, additional non-encapsulated antiperspirant agents and other components known in the state of the art for use in topical antiperspirant and deodorant compositions.

In an embodiment, optionally in combination with one or more features of the various embodiments described above or below, the composition further comprises one or more additional deodorant active ingredients. The term "deodorant" active ingredient refers to any topical material that is known or otherwise effective in preventing or eliminating malodor associated with perspiration. Suitable deodorant active agents may be selected from the group consisting of antimicrobial agents (e.g., bacteriocides, fungicides), malodor-absorbing material, and mixture thereof. For example, antimicrobial agents may comprise cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmethyl sarcosine, lauroyl sarcosine, N-myristoyl glycine, potassium N-lauryl sarcosine, trimethyl ammonium chloride, sodium aluminum chlorohydroxy lactate, triethyl citrate, tricetylmethyl ammonium chloride, 2,4,4'-trichloro-2'-hydroxy diphenyl ether, 3,4,4'-trichlorocarbanilide , diaminoalkyl amides such as L-lysine hexadecyl amide, heavy metal salts of citrate, salicylate, and piroctose, especially zinc salts, and acids thereof, heavy metal salts of pyrithione, especially zinc pyrithione, zinc phenolsulfate, farnesol, and mixture thereof. The amount of deodorant active ingredient can be comprised from 0.001 % to 2% by weight.

In an embodiment, optionally in combination with one or more features of the various embodiments described above or below, the composition further comprises one or more additional non-encapsulated antiperspirant active ingredients. For the purposes of the invention, the term "non-encapsulated antiperspirant" active ingredient refers to any compound having antiperspirant activity that is forming part of the pharmaceutical or cosmetic composition being outside the microcapsules of the invention. All the embodiments disclosed above for the "encapsulated antiperspirant active ingredients" of the invention applies also for the additional "non-encapsulated antiperspirant active ingredients". In an embodiment, optionally in combination with one or more features of the various embodiments described above or below, the non-encapsulated antiperspirant active ingredient as defined above is an aluminum-containing salt, a zirconium-containing salt and mixture thereof; more preferably the non-encapsulated antiperspirant active ingredient as defined above is an aluminum-containing is an aluminum-containing salt.

In an embodiment, optionally in combination with one or more features of the various embodiments described above or below, the weight ratio between the amount of the additional non-encapsulated antiperspirant active ingredient and the encapsulated antiperspirant active ingredient is comprised from 4:1 to 1:2; preferably the weight ratio is comprised from 2.1 to 1:1. In an embodiment, optionally in combination with one or more features of the various embodiments described above or below, the amount of additional non-antiperspirant active ingredient is comprised from 10% to 40% by weight of the weight of the encapsulated antiperspirant active ingredient. These compositions are advantageous because are non-irritant compositions that have a dual effect, a first inmediate antitranspirant effect due to the present of the additional non-encapsulated active ingredient and a long lasting antiperspirant effect due to the genipin cross-linked alginate-chitosan microcapsules of the present invention which comprises the encapsulated antiperspirant active ingredient.

The genipin cross-linked alginate-chitosan microcapsule as defined above or alternatively, the topical pharmaceutical composition which contains the microcapsules of the invention can be used for topical application to the skin for use in the prophylaxis and/or treatment of a disease or condition which transcur through an excessive or abnormal perspiration. Disease or conditions which transcur through an excessive or abnormal perspiration include hyperhidrosis, chromhidrosis and bromhidrosis. Thus, another aspect of the present invention is a genipin cross-linked alginate-chitosan microcapsule or alternatively of a topical pharmaceutical composition as defined above, for use in the prophylaxis and/or treatment of a disease or condition which transcur through an excessive or abnormal perspiration. This aspect could be also formulated as the use of the genipin cross-linked alginate-chitosan microcapsule or alternatively of a topical pharmaceutical composition as defined above for the preparation of a medicament for the prophylaxis and/or treatment of a disease or condition which transcur through an excessive or abnormal perspiration. It also relates to a method for the prophylaxis and/or treatment of a mammal suffering or is susceptible to suffer from a disease or condition which transcur through an excessive or abnormal perspiration, the method comprises administering to said mammal an effective amount of the genipin cross-linked alginate-chitosan microcapsule, or alternatively of a the topical pharmaceutical composition of the present invention. Thus, in a preferred embodiment, it is provided the genipin cross-linked alginate-chitosan

microcapsule, or alternatively of a topical pharmaceutical composition as defined above for use in the prophylaxis and/or treatment of hyperhidrosis.

The genipin cross-linked alginate-chitosan microcapsule as defined above or alternatively, the topical cosmetic composition of the invention can be used for reducing the normal perspiration of the skin. Thus, another aspect of the present invention is a use of the genipin cross-linked alginate-chitosan microcapsule or alternatively of a topical cosmetic composition as defined above as antierspirant and deodorant agent. The genipin cross-linked alginate-chitosan microcapsule as defined above or alternatively, the topical cosmetic composition of the invention reduces the extent of sweating, the occurrence of unsightly sweat marks on clothing as well as minimizing body odour. The topical cosmetic composition of the present invention is designed to apply to the body to improve its appearance or to beautify, preserve, cleanse and protect the skin. Therefore, the above cosmetic compositions are adjectivally used for a non-medical application.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

EXAMPLES

**1. Genipin cross-linked alginate-chitosan microcapsules**

**1.1. Microcapsules of the invention**

**1.1.A. Composition**

Table 1 shows the qualitative and quantitative composition of the genipin cross-linked alginate-chitosan microcapsules of the present invention. The amount of each ingredient is expressed in weight percent in relation to the total weight of the composition.

Table 1

| | **Microcapsule Example 1** | **Microcapsule Example 2** | **Microcapsule Example 3** |
|---|---|---|---|
| Quitosan | 2.4 | 3.2 | 2.7 |
| Alginate | 1.2 | 1.6 | 1.4 |
| Genipin | $4\times10^{-3}$ | $6\times10^{-3}$ | $2\times10^{-2}$ |
| Aluminum-containing salt | 96.4 | 95.2 | 95.9 |
| Crosslinking % | 34.6 | 34.3 | 34.0 |
| weight ratio chitosan/ alginate | (2:1) | (2:1) | (2:1) |
| weight ratio chitosan/ aluminum-containing salt | (1:40) | (1:30) | (1:35) |
| D90 (μm) | 60 | 30 | 20 |
| D50 (μm) | 12 | 10 | 10 |

**1.1.B. Preparation process**

The genipin cross-linked alginate-chitosan microcapsules of the present invention disclosed in section 1.1.A. were prepared by spray drying an aqueous dispersion and the process comprises the following steps:

<u>Preparation of Microcapsules of Example 1</u>

(a) Preparation of solution A: In a beaker with 100 mL of acidic aqueous solution (1g of acetic acid in 100mL water) 1g of chitosan was added and the resulting mixture was stirred for 1 hour at 1000rpm in a IKA stirrer with propeller-shaped blades. To the resulting mixture, 40g of aluminum-containing salt were added and it was stirred for an additional hour at the same speed (1000rpm) to obtain solution A.

(b) Preparation of solution B: In a beaker with 100mL of water 0.5g of alginate and 4mL of genipin 2mM were dispersed to obtain solution B.

(c) Solution B was dropped onto the solution A and the resulting solution was stirred for 5 hours at a speed of 500rpm to obtain solution C.

(d) The Solution C was pumped at a rate of 3mL/min into a co-current spray dryer (Buchi) and atomized using a two-fluid nozzle. The spray drying were carried out with an air flow of 473L/h, a feed flow of 3mL/min, an inlet air temperature of 200°C, and an outlet air temperature of 104°C. The dried powder obtained (microcapsules of Example 1) was collected at the bottom of a cyclone.

Preparation of Microcapsules of Example 2 and 3

Microcapsules of Example 2 and 3 disclosed in the section 1.1.A. were prepared following the same process as defined for microcapsules of Example 1 using the following amount of the components:

Microcapsules of Example 2: 100mL of acidic aqueous solution (1 g of acetic acid in 100mL water); 1g of chitosan; 30g of alumminum-containing salt; 100mL of water; 0.5g of alginate and 4ml of genipin 2mM.

Microcapsules of Example 3: 100mL of acidic aqueous solution (1g of acetic acid in 100mL water); 1g of chitosan; 35g of alumminum-containing salt; 100mL of water; 0.5g of alginate and 14ml of genipin 2mM.

**1.2. Comparative microcapsules**

**1.2.A. Composition**

Table 2 shows the qualitative and quantitative composition of the genipin cross-linked alginate-chitosan microcapsules having a percentage of crosslinking outside the scope of the present invention.

Table 2

| | **Microcapsule comparative Example 1** | **Microcapsule comparative Example 2** |
|---|---|---|
| Quitosane | 2.4 | 2.4 |
| Alginate | 1.2 | 1.2 |
| Genipin | 0.001 | 0.9 |
| Aluminum-containing salt | 96.4 | 95.5 |
| Crosslinking % | **10** | **45** |
| weight ratio chitosan/ alginate | (2:1) | (2:1) |
| weight ratio chitosan/ aluminum-containing salt | (1:40) | (1:40) |
| D90 (μm) | 30 | 30 |
| D50 (μm) | 10 | 5 |

**1.2.B. Preparation process**

The comparative Examples 1 and 2 of genipin cross-linked alginate-chitosan microcapsules disclosed in section 1.2.A. were prepared following the same process as defined in section 1.1.B. using the following amount of the components:

Microcapsules of Comparative Example 1: 100mL of acidic aqueous solution (1g of acetic acid in 100mL water); 1g of chitosan; 40g of aluminum-containing salt; 100mL of water; 0.5g of alginate; and 0.5mL of genipin 2mM.
Microcapsules of Comparative Example 2: 100mL of acidic aqueous solution (1g of acetic acid in 100mL water); 1g of chitosan; 40g of aluminum-containing salt; 100mL of water; 0.5g of alginate; and 0.8L of genipin 2mM.

## 2. Antiperspirant and deodorant compositions

### 2.1.A Composition

Table 3 shows the qualitative and quantitative composition of antiperspirant and deodorant compositions of the present invention and comparative compositions of the invention in form of spray.

Table 3

| INCI Name | Spray Composition Example 1 | Spray comparative Composition Example 1 | Spray comparative Composition Example 2 |
|---|---|---|---|
| | Weight (%) | | |
| genipin cross-linked alginate chitosan microcapsules[(a)] | 6% Microcapsules Ex. 1 | 6% Microcapsule s comparative Ex. 1 | 6% Microcapsule s comparative Ex. 2 |
| Aluminium Sesquichlorohydrate | 14 | 14 | 14 |
| Disterdimonium Hectorite | 20 | 20 | 20 |
| Perfume (Fragrance) | 6.67 | 6.67 | 6.67 |
| Cyclopentasiloxane | q.s. 100 | q.s. 100 | q.s. 100 |
| (a) The amount of 6% by weight of the microcapsules in relation to the total weight of the composition corresponds to an amount of encapsulated aluminum-containing salts of 2.8% by weight of the total weight of the composition. q.s.: quantity sufficient to complete 100 g of topical composition | | | |

Table 4 shows the qualitative and quantitative composition of antiperspirant and deodorant compositions of the present invention and comparative compositions of the invention in form of roll-on.

Table 4

| INCI Name | Roll-on Composition Example 2 | Roll-on comparative Composition Example 3 | Roll-on comparative Composition Example 4 |
|---|---|---|---|
| | Weight (%) | | |
| Phase A | | | |
| PPG-15 Stearyl Ether | 2 | 2 | 2 |
| STEARETH-21 | 1.5 | 1.5 | 1.5 |
| STEARETH-2 | 3 | 3 | 3 |
| Phase B | | | |
| genipin cross-linked alginate chitosan microcapsules | 4.2 % Microcapsules Ex. 1 | 4.2% Microcapsule s comparative Ex. 1 | 4.2% Microcapsule s comparative Ex. 2 |
| Aluminium Sesquichlorohydrate | 24.5 | 24.5 | 24.5 |

(continued)

| INCI Name | Roll-on Composition Example 2 | Roll-on comparative Composition Example 3 | Roll-on comparative Composition Example 4 |
|---|---|---|---|
| | Weight (%) | | |
| **perfume (Fragrance)** | 1.35 | 1.35 | 1.35 |
| (a) The amount of 4.2% by weight of the microcapsules in relation to the total weight of the composition corresponds to an amount of encapsulated aluminum-containing salt of 1.68% by weight of the total weight of the composition.<br>q.s.: quantity sufficient to complete 100 g of topical composition | | | |

**2.1.B. Preparation process**

Spray compositions

The antiperspirant and deodorant compositions in form of spray (Composition Example 1 of the present invention and comparative Composition Example 1 and 2) disclosed in section 2.1.A were prepared following the process which comprises:

Cyclopentasiloxane and 20 g of disterdimonium Hectorite into a mixer while stirring until complete dispersion. Then, add 6.6g of perfume and 14g of an aluminum-containing salt and the microcapsules of Example 1, or comparative Example 1 or comparartive Example 2. 25% by weight of the obtained dispersion was loaded into an aerosol container and charged with 85% by weight of the propellant.

Roll-on compositions

The antiperspirant and deodorant compositions in form of roll-on (Composition Example 2 of the present invention and comparative Compositions Example 3 and 4) disclosed in section 2.1.A were prepared following the process which comprises:

Mixing all the ingredients of phase A into water heated at 75°C. Once all the ingredients are loaded, emulsify the mixture during 5 minutes at 60Hz. Then, the resulting emulsion is colled while stirring at 30Hz. The resulting mixture is cooled to at 45°C while strirring and adding all ingredients of phase B one after the other. Then, stiiring at 30°C until the viscosity increase.

**3. Stability test**

The presence of microcapsules was monitored using a fluorescence-equipped upright microscope and a scanning electronical microscope.

For the microcapsules, three measurements were taken on the same sample at three diferent temperatures (i.e. 4°C, 25°C and 40°C) every month during three months. In the case of the antiperspirant and deodorant compositions, three measurements were taken on the same sample at three diferent temperatures (i.e. 4°C, 25°C and 40°C) every month during three months for the roll-on compositions and every month during six months for the spray compositions.

**Methods**

**A. Optical microscopy with fluorescence**

The reaction of chitosan with genipin generates a fluorescent compound at a wavelengths emission-excitation of 369 and 470 nm respectively.

Equipment: Leica TCS SP2, Germany.

Process: 0.5mg of the microcapsules of Examples 1 or 2 of the present invention (cf. section 1.1.A) or alternatively 0.5mg of the microcapsules of comparative Examples 1 and 2 outside the scope of the present invention (cf. section 1.2.A) were placed on a glass slide and the microparticles were measured in at least three different areas of observation.

Conditions:

wavelength (λ)= 470nm
Temperature = 25°C
Magnifications = 40X and 100X.

**B. Scanning Electronical Microscopy**

Equipment: JEOL, JSM-840.

Process: 0.5mg of the microcapsules of Examples 1 or 2 of the present invention (cf. section 1.1.A) or alternatively 0.5mg of the microcapsules of comparative Examples 1 and 2 outside the scope of the present invention (cf. section 1.2.A) were dried in an oven under vacuum overnight.

The dried microcapsules were mounted on the holders using double-sided conductive tapes. The powdered dried microcapsules were sprinkled lightly with a spatula and pressed lightly to seat. Then, the microcapsules were holded upside down and were tapped it. Finally, microcapsules were fixed in stubs using double-faced copper adhesive tape and coated with gold for enhancing conductivity before scanning to obtain a coating of 10nm of thickness using the Cressington 108 sputter coater. The sputtering process inluded two steps: 1.Vacuum of 0.1 Pa to remove water and oxygene molecular that might damage the surface sample and 2. the metal deposition under this pression and a current 20mA.

Conditions: vacuum (in the sample chamber $10^{-4}$ Pa)
Magnifications= 500X, 1000X and 3000X.

**3.1. Microcapsules**

As it is shown in Fig. 2A, the microcapsules of Example 1 of the present invention which are formed by genipin cross-linked alginate-chitosan microcapsule comprising one or more aluminum-containing salts and a percentage of croslinking equal to or higher than 20% expressed in absorbance values were stable even when they were submitted during three months at 40°C.

In comparison, as it is shown in Fig. 2B, the microcapsules of comparative Example 1 which has a percentage of crosslinking below the claimed range (i.e 10% by weight) were easily broken even when they submitted during 1 months at 25°C.

**3.2. Antiperspirant and deodorant compositions**

The antiperspirant and deodorant composition of Example 1 of the present invention which is in form of spray and comprises the microcapsules of Example 1 were stable even when they were submitted during six months at 40°C. Equally, the antiperspirant and deodorant composition of Example 2 of the present invention which is in form of roll-on and comprises the microcapsules of Example 1 were stable even when they were submitted during three months at 40°C.

In comparison, the antiperspirant and deodorant comparative compositions of Example 1 and 3 which are in form of spray and roll-on respectively and comprises the microcapsules of comparative Example 1 which has a percentage of crosslinking below the claimed range (i.e 10% by weight) were easily broken even when they submitted during 1 months at 25°C.

**4. Release test- antiperspirant Effect test of the Antiperspirant and deodorant compositions**

**Process**

The antiperspirant and deodorant spray compositions of Example 1 of the present invention; or the antiperspirant and deodorant roll-on compositions of Example 2; or the antiperspirant and deodorant spray comparative Example 2 outside the scope of the present invention; or the antiperspirant and deodorant roll-on comparative Example 4 outside the scope of the present invention were applied in one armpit and the other armpit was used as control. The left or right

armpit was randomly selected (50% of the volunteers had the product on the right, the other 50% on the left). The test consists on the use of the tested compositions during one week for the evaluation of the 72 hours antiperspirant effect.

**Method**

At baseline, the evaluation was performed by gravimetry, weighting the sweat. After 7 days using the tested composition, the evaluation was performed by the technicians, at time 24, 48 and 72 hours after a single application. The parameter evaluated was the weight of the sweat.

The efficacy of the composition that was tested in both armpits, after being used in normal usage conditions, has been evaluated by extrapolation of the results which were obtained under these particular experimental conditions.

The study was carried out following general conditions in an external laboratory, established for the execution of study project on humans.(Structure and Content of Clinical Study Reports from ICH Harmonised Tripartite Guideline; International Recommendations ICH Topic E6, CPMP/ICH/135/95 of May 1st 1996, European Parliament and Council Guideline 2001/20/CE - DOCE OF May 1st 2001).

Inclusion criteria

The specific inclusion criteria, defined in the protocol, were as follow:

- Age: 18-70 years old
- Sex: both
- Photo-type (Fitzpatrick): I to VI
- All types of skin.

Non-inclusion criteria

The specific non-inclusion criteria, defined in the protocol, were as follow:

- Injuries or infection in the experimental area.
- Pathologies in the experimental area.
- Eczematous reaction which has not fully disappeared, scar or pigmentation complications from previous studies in the experimental area.
- Intense sun or UV rays exposure during the study or during the previous month to the study.
- Treatment with any medicine for psoriasis, vitiligo, within one month before the study.
- Allergies to cosmetics products, frequently.
- Cutaneous hyper-reactivity.
- Atopic dermatitis.
- Being pregnant or breastfeeding.
- Armpits not depilated.

Criteria for application the product

Experimental area: Armpit (The left or right armpit was randomly selected by the researcher).
Tested composition: The tested composition was directly applied on the armpit skin of the volunteer.
Applied quantity: 1ml of the tested composition.

Experimental procedure

The first day of the test, instructions of the study were given to the volunteers. Before starting the study they filled the informed consent and the exclusion criteria. The tested composition was used by the volunteers during a week. The eighth day the product was applied by the researcher in charge at the center. During the next three days the volunteers were instructed to not use any deodorant.

The evaluation was performed measuring the weight of the sweat the first day of the study and after the eighth day, it was measure 24 hours, 48 hours and 72 hours after this last application.

Chronology of the study

Duration of the study: 11 consecutive days
Application of the tested composition:

At home Days 1, 2, 3, 4, 5, 6 and 7.
At the center Day 8.

Sweat Weight: after the tested composition application (day 8); at 24 hours (day 9); at 48hours (day 10); at 72 hours (day 11).

Day 1: Baseline

1. The volunteer washed its armpits with sponges impregnated of neutral soap without parfum.
2. The researcher weighed absorbent discs before putting them in contact to the clean armpits of each volunteer.
3. The volunteers remained in a room at 30°C (30-40% relative humidity(RH)) during 40 minutes.
4. After these 40 minutes, the absorbent discs were placed on both armpits of each volunteer.
5. The volunteers remained another 20 minutes in a room at 30°C (30-40% RH).
6. The discs were weighed again after the volunteers had been sweating.
7. The armpits were cleaned again.
8. The tested composition was applied on one armpit of each volunteer by the researcher.
9. The volunteers were instructed to use the tested composition during 7 days at home, once per day, only in the armpit selected by the researcher.

Day 8:

1. The experimental areas were cleaned using neutral soap in the center.
2. The tested composition was applied over the treated armpit of each volunteer by the researcher.
3. The volunteers were instructed to not apply the tested composition during the following 72 hours.

Day 8 + 24, 48 and 72 hours (Antiperspirant Evaluation)

1. The researcher weighed absorbent discs before putting them in contact to the armpits of each volunteer.
2. The volunteers remained in a room at 30°C (30-40% RH) during 40 minutes.
3. After these 40 minutes, the absorbent discs were placed on both armpits of each volunteer.
4. The volunteers remained another 20 minutes in a room at 30°C (30-40% RH).
5. The discs were weighed again after the volunteers had been sweating.

Gravimetry

To determinate the amount of sweat a Precision Balance Model PS 750 R2 (Radwag) was used. The procedure to evaluate the amount of sweat for each volunteer, at the different days of control, was the following one:

First of all, the researcher weighed the absorbent disc. After the first 40 minutes, the disc was place on the armpit of the volunteer. After the volunteers had been sweating during 20 minutes, the absorbent discs were weighed again in order to determine the amount of sweat

Statistical Analysis

In order to analyse the results it was carried out a statistical analysis before and after the treatment, including

normal behaviour of the variables, Saphiro-Wilk test, non-parametric test and Wilcoxon test:

Shapiro-Wilk Normality test: This test uses the "null hypothesis" principle in order to analyse the normality of the variables. According to this test it was concluded that the variables do not come from a normally distributed population. Therefore it was applied a non-parametric test.

Wilcoxon test: This test is a non-parametric statistical hypothesis test used when comparing two related samples. It has been used in order to analyse the effect of the treatment over time, compared to the evolution of the sweat on the control armpit. The significance value established was 0.05 (95% confidence interval). Statistical analysis allows us to see if the product reduces the armpit sweat compared to the control.

**Results**

The compositions as defined above which comprises the microcapsules of the present invention exhibit an antiperspirant effect at 24 hours and at 48 hours. Besides, these compositions also reduce the sweat at 72 hours after their application.

On the other hand, the antiperspirant and deodorant comparative compositions of Example 2 and 4 which are in form of spray and roll-on respectively and comprise the microcapsules of comparative Example 2 which has a percentage of crosslinking above the claimed range (i.e. 45% by weight) were stable even when they were submitted during three months at 40°C. However, they do not exhibit the appropriate release of the aluminum-containing salts from the microcapsules for being used as a long-lasting antiperspirant and deodorant composition. In particular, the antiperspirant and deodorant comparative compositions of Example 2 and 4 do not exhibit an antiperspirant effect at 24 hours after their application. However, they exhibit antiperspirant effect and reduction of the sweat at least from 48 hours to 72 hours.

Thus, only the compositions of the invention which comprises the genipin cross-linked alginate-chitosan microcapsules of the present invention having a percentage of crosslinking comprised from 20 to 40% are stable and exhibit the appropriate sustained antiperspirant effect.

**5. Cumulative skin irritation potential**

**Cytotoxicity test**

The possible cytotoxic effect of the genipin cross-linked alginate-chitosan microcapsules of Example 1 of the present invention or the cytotoxic effect of non-encapsulated aluminium sesquichlorohydrate salts on skin cells were evaluated.

Method

Human Epidermal Keratinocytes (HPEKp) were repeatedly treated with eight different concentrations of the genipin cross-linked alginate-chitosan microcapsules of Example 1 and the non-encapsulated aluminium sesquichlorohydrate salts (from 0.0015% to 0.2% by weight of the total weight of the solution) for 72hours for 3 consecutive days with an application of the tested products each 24h.

Cell viability was determined by Resazurin test at the end of each 24 hours treatment (that is 24 hours, 48 hours and 72 hours). Maximum non-cytotoxic concentration of the test products on the skin cells was defined based on the results obtained in this study.

Then, cells were exposed to the following concentrations of each test products: 0.006%, 0.003% and 0.0015%,

Procedure:

HEPc (human epidermal keratinocytes) were seeded in 96-well culture plate and maintained in CnT-Prime medium (Growth medium) until a cell confluence of 75-80% (48 - 72h after seeding). Thereafter, they were treated with serial dilutions of the test products (0.2, 0.1, 0.05, 0.025, 0.012, 0.006, 0.003 and 0.0015%) prepared in keratinocyte maintenance medium (Application 1). After 24h of treatment, test solutions as mentioned above were removed and cultures were washed twice with phosphate buffered saline (PBS) solution, in order to complete eliminate the product remaining. Subsequently, the cell viability was evaluated by resazurin test. After this test, serial dilutions of the test products were again prepared and cells exposed for an additional 24h (Application 2). After 24h from the second application, cell viability was evaluated again. A third preparation and application of the serial dilutions of the test products was performed (Application 3) and cell viability was evaluated again after 24h from this third application.

Non-treated cells and 0.02% Sodium Dodecyl sulfate (SDS)-treated cells were used as negative and positive

controls, respectively. Each experimental condition was evaluated in triplicate.

Resazurin test:

The commercial available resazurin Alamar Blue® (Invitrogen) was used. Alamar Blue® is a fluorometric/colorimetric growth indicator based on detection of cell metabolic activity. Specifically, this system is an oxidation-reduction (REDOX) indicator that both fluoresces and changes color in response to chemical reduction of growth medium resulting from cell growth. Cell innate metabolic activity produces a chemical reduction of the Alamar Blue® dye. Continued growth maintains a reduced environment while inhibition of growth maintains an oxidized environment. Reduction related to growth causes the REDOX indicator to change from oxidized (non-fluorescent, blue) form to reduced (fluorescent, red) form, which can be measured by fluorometric or spectrophotometric analysis. Alamar Blue® was applied to the cell culture at 10% (v/v) in growth medium. After 2h of incubation (37°C, 5%CO2), fluorescence was determined in a fluorometer at 540nm excitation wavelength and 590nm emission wavelength. Data was properly recorded for further processing.

Data analysis:

Analysis of data from the three tested culture plates (one plate for each evaluated exposure period; 24, 48 and 72h) was performed. Mean relative fluorescence units (MRFU) and standard deviation (SD) was calculated for treated and non-treated cells before (baseline condition) and after each product application (Application 1, 2 and 3). To normalize relative fluorescence values from the different applications plates, average of all the well values before application (baseline condition) of each plate was calculated and thereby, a correction factor per well was determined, which was used to calculate cell viability in the three consecutive exposures times. This correlation factor allowed the calculation of the MRFU with the replicates from the different evaluated plates for each experimental condition. For each evaluated exposure time, the cell viability of non-treated cells was considered as 100% and viability of the treated-cells was calculated relative to this value.

**<u>IL-1α and IL-8 release assessment on human keratinocytes:</u>**

This test is intended to verify that repeated exposure to products not induce an inflammatory response in human keratinocytes. To this aim, human keratinocytes were exposed to the test products for 3 days with a product application each 24h, then the release of the inflammatory mediators IL-1α (primary cytokine constitutively produced in epidermal keratinocytes, which is accumulated intracellularly and released in response to skin irritation processes) and IL-8 (potent chemotactic and proinflammatory cytokine) were determined in treated and non-treated cells (control).

Procedure:

For this assay, skin cells (human keratinocytes) were plated in 24-well culture plate and maintained in growth conditions (37°C, 5%CO2) until 60-70% cell confluence (48-72h after cell seeding). Then, the growth medium was replaced with maintenance medium and keratinocytes were maintained in this medium for an additional 18-20 hours. After that, cells were exposed to 3 concentrations of the test products (0.006%, 0.003% and 0.0015%), 3 consecutively times with one application every 24 hours (Treatment total duration: 72h). Cell culture supernatants were collected after 22h from each application and stored at -80°C for the subsequent quantification of the released inflammatory mediators. After supernatant collection, cultures were washed twice with PBS and the vital dye Alamar Blue® was added to cell viability evaluation (cf. Cytotoxicity test).

IL-1α and IL-8 quantification:

Quantification of IL-1α and IL-8 in the collected cell supernatants were performed by Enzyme-linked immunosorbent assay (ELISA), using specific and highly sensitive primary antibodies for the recognition of these inflammatory mediators. This system requires the use of a different ELISA kit for each mediator to be quantified (ELH-IL1A and ELH-IL8, RayBiotech). Non-treated cells and 4μg/ml Retinoic Acid (RA)-treated cells were used as negative and positive controls, respectively. Since RA was dissolved in DMSO (dimethylsulfoxide) for its application to the cultures, a vehicle control was included in the assay (0.125% DMSO-treated cells). Each experimental condition was evaluated in triplicate.

Analysis data:

A standard curve for each cytokine (human IL-1α and IL-8) was evaluated in each performed ELISA. The

standard curve was determined by plotting the obtained absorbance values on the y-axis and concentration on the x-axis. Then, the best-fit straight line was drawn through the standard points (linear regression). IL-1α and IL-8 concentration on analysed cell supernatants was determined by interpretation from the standard curve and expressed in pg/ml. The fold increase in the concentration of each cytokine in treated cells compared to untreated was calculated for each exposure time and evaluated product. A ratio IL-8/IL-1α was calculated for each experimental condition from the obtained fold increase values.

**5.1. Results**

A ratio IL-8/IL-1α was calculated for each test product as defined above. Results indicate that the genipin cross-linked alginate-chitosan microcapsules of the present invention, which comprise encapsulated aluminium-containing salts are less irritants than the non-encapsulated aluminium sesquichlorohydrate salts.

The release of IL-1α is the first event in the cascade of inflammation in response to an irritant compound. This cytokine stimulates production and release of more IL-1 α and IL-8. It has been observed that the ratio IL-8 / IL-1 α after exposure to irritants is less than 1 (Coquette et al., 2003, Poumay et al., 2004).

Ratios of IL-8/IL-1α after application of the tested non-encapsulated aluminium sesquichlorohydrate salt decrease between the first and the third application of each concentration and between concentrations. A more exposures and the higher the concentration, ratio is closer to values of irritating compounds.

However, the ratios of IL-8/IL-1α after application of the genipin cross-linked alginate-chitosan microcapsules of the present invention, which comprise encapsulated aluminium-containing salts decrease with increasing concentration. The second application of the two lower concentrations increases the ratio by increased release of IL-8.

The IL8 / IL-retinoic 1α ratios are less than 1 (typical irritating products) after the second and third application. Those ratios of the non-encapsulated aluminium-containing salts decrease with increasing concentration and repeating the application. Those ratios of genipin cross-linked alginate-chitosan microcapsules of the present invention also decrease with increasing concentration but are above those of the non-encapsulated aluminium-containing salts. Therefore, the non-encapsulated aluminium-containing salts exhibits a higher profile irritating response than the genipin cross-linked alginate-chitosan microcapsules of the present invention of the present invention.

REFERENCES CITED IN THE APPLICATION

1. Schmid, F.X. "Optical spectroscopy to characterize protein conformation and conformational changes. In Protein Structure: A Practical Approach", T.E. Creighton, ed., IRL Press, Oxford, U.K., 1997, pp. 261-297.

2. Mi, F. L., Tan, Y. C., Liang, H. C., Huang, R. N., & Sung, H. W. "In vitro evaluation of a chitosan membrane cross-linked with genipin". Journal of Biomaterials Science Polymer Edition, 2001, vol. 12(8), pp. 835-850.

## Claims

1. A genipin cross-linked alginate-chitosan microcapsule comprising an effective amount of one or more encapsulated antiperspirant active ingredients, wherein the microcapsule has a percentage of croslinking comprised from 20 to 40% expressed in absorbance values.

2. The microcapsule according to claim 1, wherein the percentage of crosslinking is comprised from 25 to 35% expressed in absorbance values.

3. The genipin cross-linked alginate-chitosan microcapsule according to any of the claims 1-2, wherein the encapsulated antiperspirant active ingredient is one or more aluminum-containing salts.

4. The genipin cross-linked alginate-chitosan microcapsule according to any of the claims 1-3, wherein:

   the amount of genipin is comprised from 0.001 % to 5 % by weight of the weight of the microcapsule;
   the amount of the alginate is comprised from 1 % to 20 % by weight of the weight of the microcapsule;
   the amount of chitosan is comprised from 1 % to 20% by weight of the weight of the microcapsule;
   the weight ratio between the chitosan and the encapsulated antiperspirant active ingredient is comprised from 1:2 to 1:50; and

the weight ratio between the chitosan and alginate is comprised from 1:1 to 4:1.

5. The genipin cross-linked alginate-chitosan microcapsule according to any of the claims 1-4, wherein:

the amount of genipin is comprised from 0.001 % to 1 % by weight of the weight of the microcapsule;
the amount of alginate is comprised from 1 % to 3% by weight of the weight of the microcapsule;
the amount of chitosan is comprised from 1.5% to 4% by weight of the weight of the microcapsule;
the weight ratio between the chitosan and the encapsulated antiperspirant active ingredient is comprised from 1:10 to 1:40; and
the weight ratio between the chitosan and alginate is comprised from 1:1 to 2:1.

6. The genipin cross-linked alginate-chitosan microcapsule according to any of the claims 1-5, wherein
the amount of genipin is 0.004% by weight of the weight of the microcapsule;
the amount of alginate is 1.2% by weight of the weight of the microcapsule;
the amount of chitosan is 2.4% by weight of the weight of the microcapsule;
the weight ratio between the chitosan and the encapsulated antiperspirant active ingredient is 1:40; and
the weight ratio between the chitosan and alginate is 2:1.

7. The genipin cross-linked alginate-chitosan microcapsule according to any of the claims 1-6, wherein the amount of encapsulated antiperspirant active ingredient is comprised from 92% to 98% by weight of the weight of the microcapsule.

8. The genipin cross-linked alginate-chitosan microcapsule according to any of the claims 1-7, which has a particle size distribution D90 comprised from 20 to 80μm.

9. A process for the preparation of the genipin cross-linked alginate-chitosan microcapsule as defined in any of the claims 1-8, which comprises:

i) preparing an acidic aqueous solution of the chitosan by contacting an acidic aqueous solution with chitosan;
ii) adding the encapsulated antiperspirant active ingredient to the solution obtained in step (i) to obtain the solution A;
iii) preparing an aqueous solution of the alginate and the genipin to obtain solution B;
iv) adding the solution B to the solution A and mixing the resulting emulsion under a period of time comprised from 1 to 6 hours to obtain a wet genipin cross-linked alginate-chitosan microcapsule comprising an effective amount of one or more encapsulated antiperspirant active ingredients; and
v) spray-drying the wet microcapsule obtained in step (iv).

10. The process according to claim 9, wherein spray drying of step (v) is performed under the following conditions:

the inlet temperature is comprised from 170°C to 210°C;
the outlet temperature is comprised from 102°C to 119°C; and
the feed flow is comprised from 2 to 15 ml/min.

11. A topical pharmaceutical or cosmetic composition comprising an effective amount of genipin cross-linked alginate-chitosan microcapsules as defined in any of the claims 1-8, together with appropriate topical pharmaceutically or cosmetically acceptable excipients or carriers.

12. The topical composition according to claim 11, further comprising an effective amount of one or more non-encapsulated antiperspirant active ingredients.

13. The topical composition according to claim 12, wherein the one or more non-encapsulated antiperspirant active ingredients are in an amount comprised from 10% to 40% by weight of the weight of the encapsulated antiperspirant active ingredients.

14. The topical composition as defined in any of the claims 11-13, wherein the topical composition is in form of a cream, powder, stick, roll-on, spray, soft solids and gels.

15. Use of the genipin cross-linked alginate-chitosan microcapsule comprising an effective amount of one or more

encapsulated antiperspirant active ingredients as defined in any of the claims 1-8, or alternatively of the topical composition as defined in any of the claims 11-14 as an antiperspirant and deodorant agent.

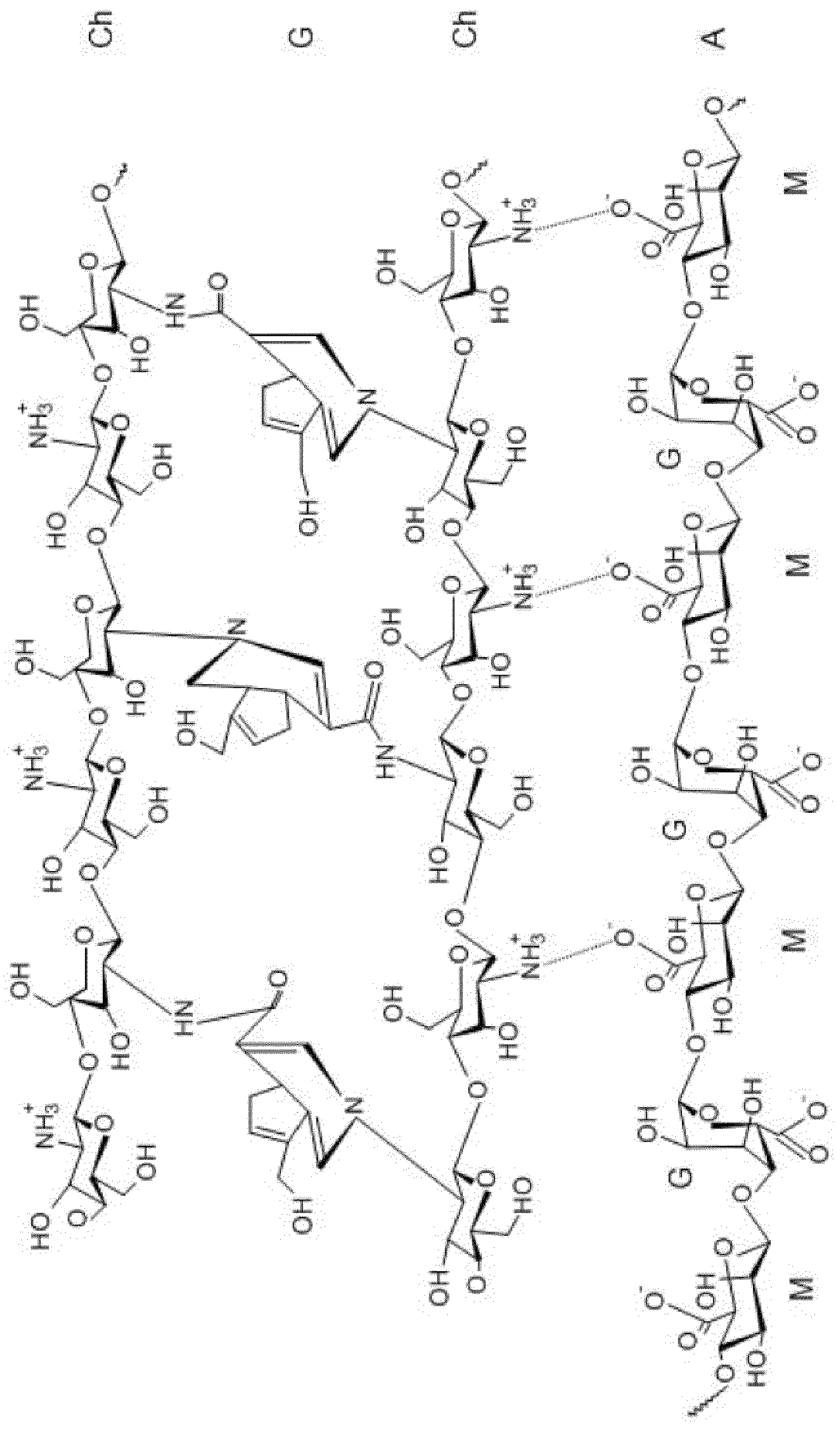

Fig. 1 (A)

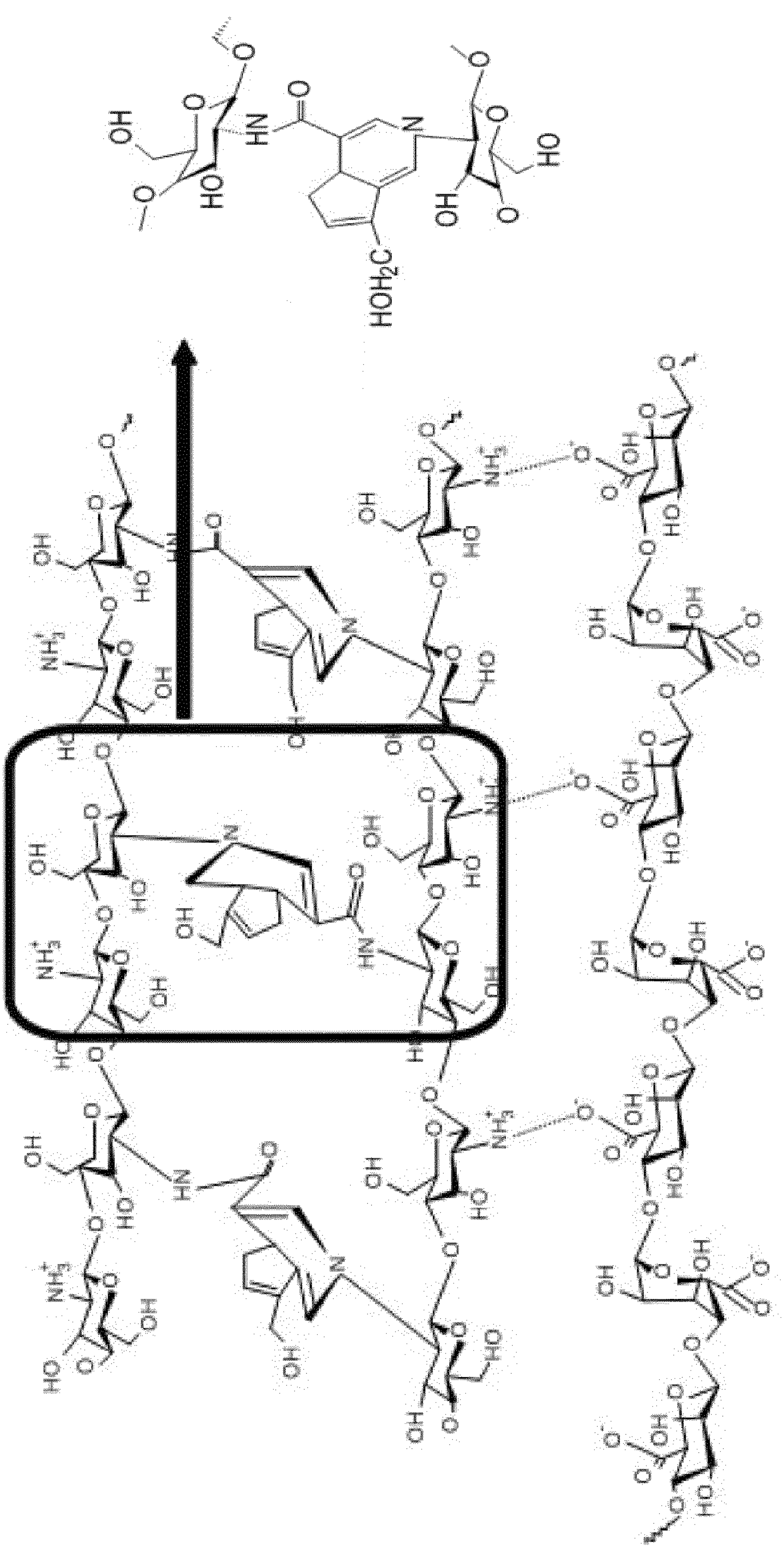

Fig. 1 (B)

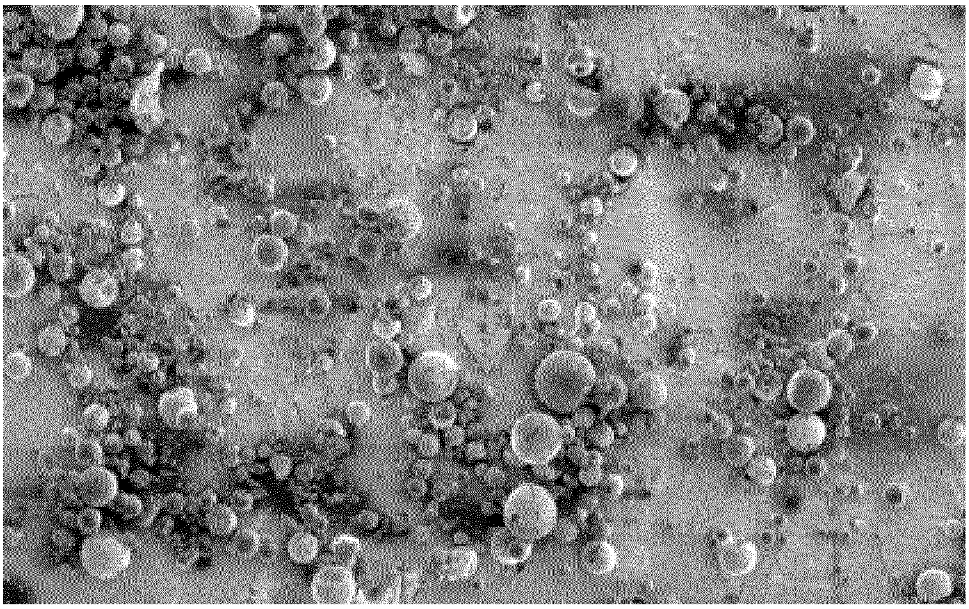

(A)

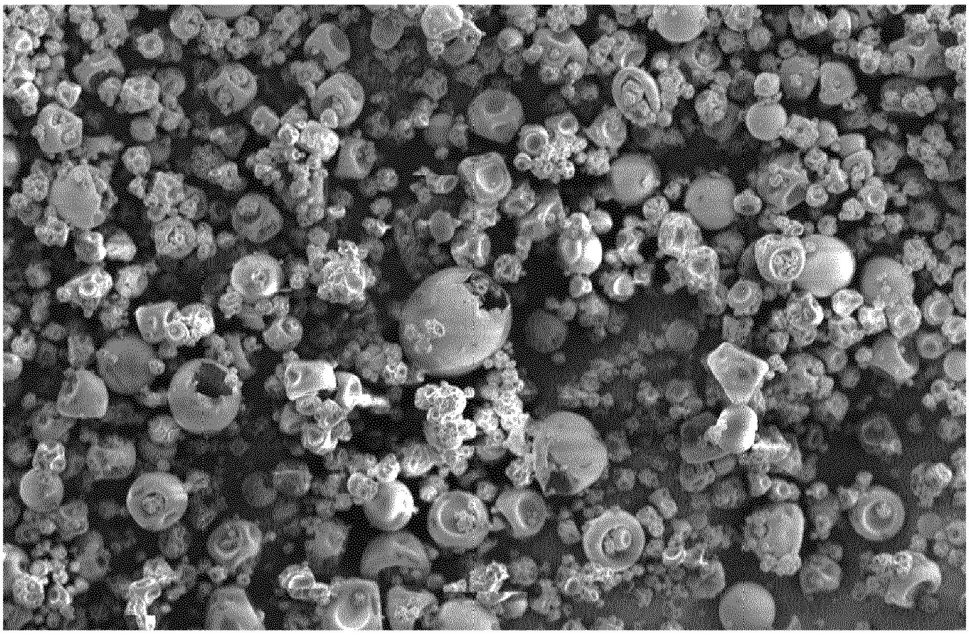

(B)

Fig.2

Europäisches Patentamt
European Patent Office
Office européen des brevets

# EUROPEAN SEARCH REPORT

Application Number
EP 16 38 2105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2003/147963 A1 (DE MORAGAS MARIA [ES] ET AL) 7 August 2003 (2003-08-07) * claims * * paragraph [0014] - paragraph [0022] * ----- | 1-15 | INV. A61K8/11 A61K8/73 A61Q15/00 |
| Y | HONGMEI CHEN ET AL: "Genipin Cross-Linked Alginate-Chitosan Microcapsules: Membrane Characterization and Optimization of Cross-Linking Reaction", BIOMACROMOLECULES, vol. 7, no. 7, 1 July 2006 (2006-07-01), pages 2091-2098, XP055136872, ISSN: 1525-7797, DOI: 10.1021/bm050862y * the whole document * ----- | 1-15 | |
| A | US 6 916 465 B2 (PANZER CLAUDIA [DE] ET AL) 12 July 2005 (2005-07-12) * claims 1-5 * ----- | 1-15 | |
| A,D | MI F-L ET AL: "IN VITRO EVALUATION OF A CHITOSAN MEMBRANE CROSS-LINKED WITH GENIPIN", JOURNAL OF BIOMATERIALS SCIENCE. POLYMER EDITION, VSP, UTRECHT, NL, vol. 12, no. 8, 1 January 2001 (2001-01-01), pages 835-850, XP008050815, ISSN: 0920-5063, DOI: 10.1163/156856201753113051 * the whole document * ----- | 1-15 | TECHNICAL FIELDS SEARCHED (IPC): A61K A61Q |

The present search report has been drawn up for all claims

1

| Place of search | Date of completion of the search | Examiner |
|---|---|---|
| Munich | 24 June 2016 | Cismaru, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 16 38 2105

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2016

| Patent document cited in search report | Publication date | Patent family member(s) | Publication date |
|---|---|---|---|
| US 2003147963 A1 | 07-08-2003 | AT 301986 T | 15-09-2005 |
| | | AU 9179201 A | 13-03-2002 |
| | | DE 50010978 D1 | 22-09-2005 |
| | | EP 1184027 A1 | 06-03-2002 |
| | | ES 2247997 T3 | 16-03-2006 |
| | | US 2003147963 A1 | 07-08-2003 |
| | | WO 0217859 A1 | 07-03-2002 |
| US 6916465 B2 | 12-07-2005 | DE 10014529 A1 | 27-09-2001 |
| | | EP 1265594 A1 | 18-12-2002 |
| | | JP 2003527408 A | 16-09-2003 |
| | | US 2003133891 A1 | 17-07-2003 |
| | | WO 0170191 A1 | 27-09-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SCHMID, F.X.** Optical spectroscopy to characterize protein conformation and conformational changes. In Protein Structure: A Practical Approach. IRL Press, 1997, 261-297
- **MI, F. L. ; TAN, Y. C. ; LIANG, H. C. ; HUANG, R. N. ; SUNG, H. W.** In vitro evaluation of a chitosan membrane cross-linked with genipin. *Journal of Biomaterials Science Polymer Edition,* 2001, vol. 12 (8), 835-850
- Structure and Content of Clinical Study Reports from ICH Harmonised Tripartite Guideline. *International Recommendations ICH Topic E6, CPMP/ICH/135/95 of May 1st 1996, European Parliament and Council Guideline 2001/20/CE - DOCE,* 01 May 2001